# EUROPEAN PATENT APPLICATION

(11) **EP 4 257 176 A1**
(43) Date of publication of application: **11.10.2023**
(21) Application number: 22166979.9
(22) Date of filing: 06.04.2022
(51) Int. Cl.: A61N 1/05, A61N 1/36

(54) **DEVICE AND METHOD FOR ELECTRICALLY STIMULATING AT LEAST ONE NERVE**

(71) Applicant: ETH Zurich, 8092 Zurich (CH)
(72) Inventor: RASPOPOVIC, Stanisa, 8006 Zürich (CH); CIOTTI, Frederico, 8048 Zürich (CH); VALLE, Giacomo, 3011 Bern (CH)

(57) **Abstract**

The invention concerns a device (1) for electrically stimulating at least one nerve (2), the device (1) comprising a bendable substrate (3) which is configured to be arranged at least partially surrounding the nerve (2) so as to define an inner surface (4) of the substrate (3) that faces an outer surface (5) of the nerve (2), and at least two electrodes (16), wherein at least one of the electrodes (16) is a surface electrode (18) and at least one of the electrodes (16) is an intraneural electrode (19), each arranged on the inner surface (4) of the substrate (3), wherein at least one surface electrode (18) is configured to establish an electric connection with a part of the nerve (2) on the outer surface (5) of the nerve (2), and wherein at least one intraneural electrode (19) is configured to penetrate the outer surface (5) of the nerve (2) to establish an electric connection with a part of the nerve (2) within the nerve (2).

The invention also concerns a method for electrically stimulating at least one nerve (2).

## Description

The invention concerns a device according to the invention, and a method for electrically stimulating at least one nerve.

Current neural interfaces have poor stimulation selectivity, high invasiveness, and low long-term stability. The effectiveness of electrical peripheral nerve stimulation has been indicated for the treatment of several conditions, such as treatment of depression and epilepsy, e.g., with vagus nerve stimulation "VNS"; amputation, e.g., with somatosensory nerve stimulation; sexual, urinary, and bowel dysfunctions, e.g., with pudendal nerve and sacral nerve stimulation; and under investigation for the treatment of cardiovascular disease. Current nerve interfaces used in clinical settings, such as helical cuffs for VNS, lack the ability to obtain selective activation of different nerve fibers while stimulating. Therefore, the current nerve interfaces lack the ability to obtain the desired physical outcomes minimizing collateral effects. Implantable devices such as transverse intrafascicular multichannel electrodes "TIME" and longitudinal intrafascicular electrodes "LIFE", have been used in small sample animal and human trials. They were able to achieve more selective stimulation but at a cost of very complex and long surgery, such as up to ten hours, together with low stability and durability, e.g., in the order of weeks or months. They rely extensively on the ability of the surgeon, hindering the clinical translation. Henceforth, in the field of bioelectronic medicine, there is a need of selective neural interface stable over time for peripheral nerve stimulation.

Neural implants have suffered from the compromise between selectivity and simplicity in manufacture and surgical implant. The unstable nature of peripheral nerves makes chronic implant of rigid electrodes such as Michigan probes and Utah arrays challenging, limiting their use mainly to the brain. Flexible electrodes specifically designed for peripheral nerve stimulation either suffer from low selectivity or inability to reach deep fascicles, or are tedious to implant, reach only a small portion of the target nerve, and are unstable, e.g., TIMEs and LIFEs. Flat interface nerve electrodes "FINEs" deform the nerve to ease the access of deeper fascicles by superficial contacts, but relying on those alone, which are distant from the target axons. This puts an upper limit to their selectivity and gives a lower efficiency at equal number of channels.

US 2007142889 A1 discloses a stimulator arrangement for stimulating nerves or other tissue includes an electrode arrangement having a substrate and a plurality of electrodes disposed on the substrate. The substrate is configured and arranged to be in a curved state prior to implantation into the body and to flatten with exposure to the body after implantation.

The problem to be solved by the present invention is to provide a device according to the invention and a method for electrically stimulating at least one nerve, which allow stimulation of nerves with easy handling.

The problem is solved by a device for electrically stimulating at least one nerve according to claim 1, and a method for electrically stimulating at least one nerve according to claim 15. Advantageous embodiments of the device are given in subclaims 2 to 14.

A first aspect of the invention is a device for electrically stimulating at least one nerve, the device comprising a substrate which is configured to be arranged at least partially surrounding the nerve so as to define an inner surface of the substrate that faces an outer surface of the nerve, and at least two electrodes, wherein at least one of the electrodes is a surface electrode and at least one of the electrodes is an intraneural electrode, each arranged on the inner surface of the substrate, wherein at least one surface electrode is configured to establish an electric connection with a part of the nerve on the outer surface of the nerve, and wherein at least one intraneural electrode is configured to penetrate the outer surface of the nerve to establish an electric connection with a part of the nerve within the nerve.

The electrically activating sites can be referred to as poles or as contacts. Also, the device can be referred to as an electrode array, wherein the activating sites can be referred to as respective electrodes of the electrode array. The inner surface of the substrate is a side of a two-dimensional surface, especially when the substrate is not yet bent, that is before arranging on a respective nerve.

The device can establish an electrode array, especially an implantable peripheral nerve electrode array, which comprises at least two electrodes. The substrate is configured to be bendable around the longitudinal direction of the device, the longitudinal direction being parallel to the axis of the nerve, when the device is placed on the nerve. The substrate can be arranged surrounding the nerve partially, for example enclosing an angle from 30° to 270°, especially 180° to 270°. The substrate can be also arranged to surround the nerve fully, i.e., the substrate enclosing an angle of 360°. The electrodes or the protrusion direction of the electrodes can be arranged orthogonal to the longitudinal direction of the device and on the inner surface of the substrate.

The surface electrode can be a conductive two-dimensional surface pad arranged on the inner surface of the substrate to come in contact with the outer surface of the nerve and configured to establish the electric connection with a part of the nerve on the outer surface of the nerve. The surface electrode can be configured to stimulate a subset of axons of the nerve. A plurality of such surface electrodes arranged orthogonal to the longitudinal direction of the device and on the inner surface of the substrate can be configured to stimulate a subset of axons at a plurality of locations. Accordingly, a plurality of such surface electrodes arranged parallel to the longitudinal direction of the device and on the inner surface of the substrate can be configured to stimulate a subset of axons at a plurality of locations in the longitudinal direction parallel to the axis of the nerve or a plurality of subsets of axons.

The device further comprises at least one intraneural electrode that is configured penetrate the outer surface of the nerve to establish an electric connection with a part of the nerve within the nerve. As a nerve comprises a bundle of a plurality of fascicles arranged parallel to the axis of the nerve and enclosed within the outer surface of the nerve, such an intraneural electrode can electrically stimulate a respective fascicle. The intraneural electrode can be arranged such that it can reach a particular predetermined fascicle and then electrically stimulate the respective fascicle. Accordingly, a plurality of such intraneural electrodes can be arranged such that they can reach one particular predetermined fascicle and then electrically stimulate the respective fascicle, or they can reach a plurality of predetermined fascicles and then electrically stimulate the respective fascicles.

Such a concurrent use of surface electrodes and intraneural electrodes to electrically stimulate at least a part of the nerve on the surface of the nerve as well at least a part of the nerve within the nerve enables a selective stimulation of the nerve. Such multipolar configurations, i.e., by means of a plurality of electrodes, can improve the spatial selectivity of the stimulation of the nerve, possibly by a combination of activating and inhibiting stimulation patterns to improve the focus of the stimulation. The electrodes can also be referred to as active sites. In other words, the presence of both intraneural and surface electrodes is essential so that deep and superficial fascicles within the nerve can be reached selectively, and so that multipolar stimulation with at least one intraneural and at least one surface electrode can be employed to inhibit untargeted areas surrounding the target area for stimulation, thereby increasing the selectivity.

The combination of surface and intraneural electrodes allows for selective activation of both superficial and deep fascicles, with reduced tissue damage compared to previous intraneural or intrafascicular electrode designs. The device according to the invention can allow for easier and faster surgical implantation than pervious intrafascicular implants found in the prior art, such as the TIME and LIFE, with more repeatable outcome, and a complete coverage of the nerve cross-section, which previously required the implant of several electrodes during a very long and complex procedure. Multiple electrodes are arranged on the single device, which can be at least partially wrapped around a nerve to activate multiple parts of the nerve. Thus, by means of the present device multiple electrodes or poles can be implanted at once for activating different parts of the nerve.

The electrical contacts are enabled by means of the electrodes. The electrodes can be arranged on respective electric traces which are connected to an implanted or an external pulse generator to provide the electrical energy. The stimulating frequency of the electric current can be below 1 kHz, typically 50 Hz or lower, especially when the intent is generation of action potentials synchronous with the stimulation. However, the stimulating frequency of the electric current can be of a higher frequency, such as between 1 kHz and 4 kHz, especially when the intent is to obtain asynchronous generation of action potentials or spatially selective stimulation by temporal interference, and such as between 4 kHz and 100 kHz, especially when the intent is to inhibit transmission of action potential along the axons.

The dimensions of the device and the electrodes can be adapted depending on the type of the nerve or the target nerve, for which the device is to be used. For example, optimal dimensions, such as the width of the device, number of electrodes, length of the needles of the intraneural electrodes which are meant to penetrate the outer surface of the nerve, or pitch between the electrodes, can be found by means of large number of computer simulations. For example, by means of the computer simulations the optimal dimensions of the device can be adapted to a pudendal nerve and a vagus nerve which have comparable size, thereby finding the best balance between device complexity and selectivity. A same or similar procedure can be re-applied to adapt the electrodes to nerves with different dimensions. In particular, the number of electrodes or the conducting channels on which the electrodes can be arranged can be chosen by the required selectivity. For example, for the aforesaid nerves of interest two alternative exemplary implementations can be proposed which lay on different points on the trade-off between complexity and performance, one with 49 electrodes and the other with 25. This proposal is a mere example and is not limited any specific number. The first alternative with 49 electrodes can reach a boundary of maximum efficiency for selective stimulation of nerve fascicles, with addition of ulterior active sites bringing diminishing improvement.

The addition of ulterior active sites bringing diminishing improvement means that for the purpose of stimulating a nerve for a particular purpose, a particular number of electrodes, in this case 49, are necessary, but further increase in the number of electrodes would not add any further benefits in terms of fascicular selectivity, as the stimulation of the nerve has been already covered and further addition would only increase the production costs of the device.

Further, in the case of the second alternative with 25 electrodes, the selectivity of the device as whole is lower than the first alternative, but the second alternative can be manufactured and implanted with ease and operated with standard equipment as compared to the first alternative.

According to an embodiment of the invention, the intraneural electrode can be a spike extending from the extending from the inner surface of the substrate and arranged to penetrate the outer surface of the nerve and configured to establish the electric connection with a part of the nerve within the nerve, the spike comprising a partially insulated part made of electrically conductive material, e.g., metal, wherein the tip of the spike is non-insulated, so that the electric connection is established with the part of a nerve within the nerve.

As a nerve is composed of plurality of fascicles, the spike upon being penetrated through the outer surface of the nerve establishes an electric connection with a corresponding fascicle. The spike extending from the inner surface of the substrate can be arranged to penetrate the outer surface of the nerve radially, so that the spike can penetrate the outer surface of the nerve causing a minimum tissue damage.

The insulated part prevents the inserted spike from establishing any electrical contact with other fascicles within the nerve which can come in contact with the surface of the inserted spike. The only electrical contact with a predetermined respective fascicle can be established by means of the tip of the spike that touches or pierces the respective fascicle. The non-insulated part of the spike can be up to 200 micron or up to 1/3rd of the length of the spike. The non-insulated part of the spike should be small enough to inject into only one fascicle and large enough to inject enough electric charge to the respective fascicle. The tip of the spike can comprise iridium oxide or platinum or Poly-3,4-ethylendioxythiophen, so that high charge injection capacity, stability and biocompatibility of the spike are enabled.

According to an embodiment of the invention, the device can be configured to supply electric current, for example from an electrical energy source such as a pulse generator, via the respective electrode to a respective part of the nerve and/or the device can be configured to receive, for example to record, a neuro-signal from a respective part of the nerve via the respective electrode. The neuro-signal can be transmitted to a recorder. In the case of a fully implanted device, the pulse generator can be an implanted pulse generator "IPG". In the case of a not fully implanted device, the pulse generator can be an external pulse generator. Furthermore, the electrodes can also be used to record neuro-signals within the nerve. The neuro-signals can be recorded as a population activity in the form of compound action potential "CAP". For example, when electric current is provided to part of the nerve by means of an electrode connected at a point or location on or within the nerve, then the corresponding reaction of the nerve to the received electric current can be recorded by means of recording the reactive neuro-signal through another electrode connected at another point or location on or within the nerve.

According to an embodiment of the invention, the substrate can comprise at least one connection element, wherein the connection element can comprise at least two electrically conducting channels, wherein at least one of the conducting channels is an electric current channel, i.e., a stimulation channel, and/or at least one of the conducting channels is a neuro-signal channel, e.g., a recording channel, each conducting channel comprising a respective electrode, wherein one end of the electric current channel can be connected or is connectable to an energy source, for example a pulse generator, which is configured to supply electric current to a part of the nerve, and/or wherein one end of the neuro-signal channel can be connected or is connectable to a recorder in order to transmit a neuro-signal from a respective part of the nerve. The electric current channel can be an electric current injection channel. The connection element can be arranged perpendicular to a longitudinal direction of the substrate, the longitudinal direction of the substrate being parallel to the axis of the nerve when the substrate is placed on the nerve. Each electrode can be arranged on a corresponding conducting channel. Through one conducting channel an electric current of respective magnitude can be conveyed. Hence, one electrode can be provided with an electric current of a magnitude which can be different to another electrode. Hence, the electrodes can be supplied with respective electric currents independent of each other. The connection element can also be used to tighten the device on the nerve.

According to an embodiment of the invention, the device can comprise a control unit that is configured to adjust a property of the electric current that can be transmitted to a respective electrode. The adjustment can be made manually or automatically by means of an algorithm. The control unit can be configured to adjust a property of the electric current that can be transmitted to a respective electrode based on the received neuro-signal from another respective electrode.

The selection of a particular magnitude of electric current that can be sent to a particular electrode can be performed by means of a control device which can facilitate a manual input which is called as an open loop procedure. This can involve a fixed stimulation policy and is simple to implement. It requires a skilled person to set parameters in the control device as per the requirements of a particular treatment procedure.

According to an embodiment of the invention, the device can comprise a recorder/ detection unit which is configured to record/detect at least one neuro-signal from at least one part of the nerve and which is connected to the control unit in order to transmit a signal corresponding to the recorded neuro-signal from at least one part of the nerve to the control unit, such that the control unit can adjust a property of the electric current that can be transmitted to a respective electrode to stimulate the at least one part of the nerve based on the at least one neuro-signal recorded/ detected by the recorder/ detection unit.

In other words, the selection of a particular magnitude of electric current that can be sent to a particular electrode can be performed by means of the control unit comprising an algorithm which is configured to stimulate the at least one part of the nerve by sending electric current and record at least one neuro-signal from at least one part of the nerve. This is procedure is referred to as a closed loop procedure. The algorithm can determine the required magnitude of electrical current that needs to be sent to a particular electrode and vary the magnitude of the electrical current that needs to be sent to the particular electrode based on the recording of the neuro-signal. In other words, the algorithm can adapt in time, in reference to the magnitude of the electric current that needs to be sent to the particular electrode. When neural implants such as electrode arrays are implanted on a nerve, then with time due to a foreign body reaction "FBR" an encapsulation of the electrode arrays can occur by means of a fibrous tissue. Furthermore, the functional outcome of a particular stimulation policy can vary due to peripheral or central adaptation. This can lead to a change in the requirement of the magnitude of the electrical current that needs to be sent to stimulate the particular part of the nerve. Hence, a closed loop procedure can lead to an efficient adaptation with time.

According to an embodiment of the invention, the substrate can comprise a central section, wherein the inner surface of the substrate corresponding to the central section can comprise a central conductive surface pad extending at least 90% of a width of the central section, the width being perpendicular to the longitudinal direction and within the plane of the substrate, wherein the longitudinal direction of the substrate is defined to be parallel to the axis of the nerve when the substrate is placed on the nerve, wherein the central conductive surface pad can be configured to establish the electric connection with a respective part of the nerve on the outer surface of the nerve. The central conductive surface pad arranged on the inner surface of the substrate corresponding to the central section may span the whole width of the device, that is in a direction perpendicular to longitudinal direction of the substrate but in the plane of the inner surface of the substrate, such that when the substrate is placed on the nerve, the longitudinal direction of the substrate is parallel to the axis of the nerve. Thus, the central conductive surface can cover the whole circumference of the nerve corresponding to the central section of the substrate.

According to an embodiment of the invention, the device can be configured to apply electric current within a frequency range between 4 kHz to 100 kHz via the central conductive surface pad, preferably between 10 kHz and 20 kHz, so that a conduction of action potential is inhibited, i.e., a virtual cut of the nerve in a section perpendicular to the axis of the nerve can be performed, in which a part of the nerve on one side of the central section in the longitudinal direction of the nerve can be treated independent of the part of the nerve on the other side of the central section. Thus, the central conductive surface pad is intended to be used for high frequency nerve block to perform a virtual cut of the nerve, and have electrodes on either side stimulating the axons independently and simultaneously in afferent and efferent directions. The central section of the device can divide the device into to symmetrical halves along the length of the nerve.

According to an embodiment of the invention, at least two electrodes are arranged on the inner surface of the substrate, wherein each of the at least two electrodes are arranged opposite to each other in a longitudinal direction of the substrate, wherein when the substrate is placed on the nerve, the longitudinal direction of the substrate is parallel to the axis of the nerve, wherein the at least two electrodes are arranged at a predetermined distance from each other, wherein the predetermined distance is at least 75% of the length of the substrate in the longitudinal direction. For example, at least one electrode can be arranged on the inner surface of the substrate proximal to one end of the substrate and at least one other electrode can be arranged on the inner surface of the substrate proximal to the other end of the substrate in a longitudinal direction of the substrate, such that when the substrate is placed on the nerve, the longitudinal direction of the substrate is parallel to the axis of the nerve. This is of advantage because the arrangement of the electrodes apart from each other by the predetermined distance, that is at least 75% of the length of the substrate in the longitudinal direction or proximal to the respective ends of the substrate along the longitudinal direction enables an optimum or maximum use of the length of the substrate, as the part of the substrate between the location of the electrode and the corresponding end of the substrate would not be in use, and hence would be not required. Hence, an optimal use of the material for the substrate can be enabled. The arrangement of the electrodes proximal to the respective two ends of the substrate enables a spatial distance between the electrodes. This ensures that the nerve is stimulated at two parts that are far enough from each other so that a selective stimulation can be enabled, as well as that a considerable difference in the stimulation of the nerve via each of the electrodes can be realized. Furthermore, the arrangement of the electrodes being at an optimal distance apart, that is large enough, or proximal to the respective two ends of the substrate enables a temporal distance between the electrodes. This means, for example, when an electric current is sent to the electrodes at one end, then the neuro-signal received from the electrodes from the other end of the substrate would enable a considerable difference in time from the generation of action potentials at the stimulating end to their arrival at the recording end, depending on the conduction velocity of each recruited axon, so to ease the removal of stimulation artifacts and allow the measurement of conduction velocity, which is characteristic for each axon and therefore can be used to identify which axons or types of axons that are being recruited.

According to an embodiment of the invention, the substrate can comprise at least two structures comprising a gap therebetween, wherein the structures are directed parallel to a longitudinal direction of the substrate, so that when the substrate is placed on the nerve, the longitudinal direction of the substrate is parallel to the axis of the nerve and that each of the gaps facilitates the bendability of the substrate when the substrate has to be arranged at least partially surrounding the nerve. Thereby, enabling the structures of the substrate to come in contact with the outer surface of the nerve. The gaps can enable the segmented substrate by means of the slotted structures to position the spikes radially around the surface of the nerve, i.e., a radial insertion of the spikes into the nerve can by thereby enabled.

According to an embodiment of the invention, the device comprises at least two structures extending from the central section in opposite directions along the longitudinal direction, wherein at least one electrode can be arranged on the inner surface of the structure. For example, at least one electrode can be arranged close to one end of the structure, whereas at least one electrode can be arranged on the inner surface of the structure close to the other end of the structure in the longitudinal direction of the substrate. The arrangement of the electrodes close to the respective two ends of the substrate enables a spatial distance as well as a temporal distance between the electrodes. In an embodiment, the device can comprise on both sides of the central section a plurality of structures spaced by a respective gap.

According to an embodiment of the invention, one surface electrode and two intraneural electrodes are arranged on the inner surface of the structure. The presence of two intraneural electrodes that are configured to simultaneously penetrate the outer surface of the nerve to establish an electric connection with a part of the nerve within nerve provides additional overall structural stability to such an electrode arrangement. This enables such intraneural electrodes from deviating upon penetrating through the outer surface of the nerve.

According to an embodiment of the invention, the substrate can be a nerve cuff comprising a flexible material for wrapping the nerve cuff around the nerve. The choice of material for the substrate is free as long as it is biocompatible and compliant enough to not damage the nerve during natural movement of the nerve. The addition of cuff-like structure improves the stability of the stimulation of the nerve, reducing the positional shift of contacts which is normally experienced with intrafascicular electrodes and is a principal cause of unwanted change of functional outcome of the stimulation. The flexible material can be polydimethylsiloxane "PDMS", so that the substrate is flexible, transparent, and biocompatible.

According to an embodiment of the invention, the substrate can comprise at least one tightening strip, so that when the substrate is placed on the nerve, the tightening strip is configured to tighten the grip of the substrate around the nerve. The tightening strip is used to tighten the device. The connection element can, however, also be used to tighten the cable as an option. The tightening strip in the middle can be used to fix the main body of the device, thereby providing mechanical stability. The dents for the tightening strip in the middle and in the ends are used as a further addition, the slots can also help a surgeon to place the strip.

The inventive device comprising multiple electrodes can enable an easier implant, as a single implant is required as compared to the multiple implants needed in case of TIMEs or LIFEs to cover the whole nerve. The device is quicker to implant than other penetrating electrodes from the state of the art, as the implantation of the device is straightforward, and a repeatable positioning and fixation of the device to the nerve is possible. The device provides a better adaptability to nerves with different diameters. Linear arrays such as TIMEs and LIFEs require the implant of an increasing number of electrodes to cover larger nerve cross-sections, requiring increasingly complex surgeries, while the inventive device can adapt by scaling up its size, thereby, maintaining or even easing the surgery. A surgical implant of the device is faster and easier than previous interfascicular electrodes such as TIMEs or LIFEs, since it requires fewer and simpler steps of placing the electrode around the nerve, fixing the electrode at the middle section, tightening the extremities on either side of the electrode.

On the other hand, cuff electrodes do not scale up well with the nerve due to the appearance of deep fascicles which are shielded from the electrode by the superficial fascicles. Furthermore, a single version of the proposed electrode can adapt to the range of a specific target nerve diameters due to natural interindividual variability. The device provides for improved spatial selectivity by multipolar stimulation between surface and intrafascicular electrodes. Further, a combination of high frequency nerve block with stimulation on a single device to obtain direction selective stimulation can be enabled by means of the inventive device. The device is more selective than current neural interfaces in clinical use. A repeatable manufacture of the device through microfabrication is possible, enabling clinical translation.

The device can be used in applications of bioelectronic medicine and neuromodulation. A potential application of the device is for the treatment of sexual dysfunctions such as erectile dysfunction and female genital arousal dysfunction. Further, the device can used as vagus nerve implant for epilepsy, depression, and cardiovascular disease treatment, and for other neurostimulation treatments such as spinal root stimulation, auditory brainstem stimulation optic nerve stimulation, and sacral nerve stimulation.

The device can be used as an implantable system for bladder/bowel control, pain treatment, sensory feedback restitution in diabatic neuropathy or in limb prostheses.

A second aspect of the invention is a method for electrically stimulating at least one nerve by means of a device according to the first aspect of the invention, the method comprising a placement of the bendable substrate on the nerve at least partially surrounding the nerve, so that the inner surface of the substrate comes in contact with the outer surface of the nerve, wherein the at least two electrodes, wherein at least one of the electrodes is a surface electrode (16) and at least one of the electrodes is an intraneural electrode, each arranged on the inner surface of the substrate to establish a respective electric contact with a respective part of the nerve, wherein at least one surface electrode establishes an electric connection with a part of the nerve on the outer surface of the nerve, and wherein at least one intraneural electrode penetrates the outer surface of the nerve to establish an electric connection with a part of the nerve within nerve.

According to an embodiment of the invention, the method can be adopted outside a human or animal body.

The invention is explained below using the embodiment examples shown in the enclosed drawings.

It is shown in
- Fig. 1:: a perspective view of an embodiment of a device according to the invention,
- Fig. 2:: a perspective view of an embodiment of a device according to the invention, wherein the device is wrapped around a nerve,
- Fig. 3:: an enlarged view of an inner surface of a substrate an embodiment of a device according to the invention,
- Fig. 4:: a schematic view of the inner surface of the device 1 of Fig. 1 and 2, and
- Fig. 5:: a schematic view of the inner surface of an embodiment of the device 1.

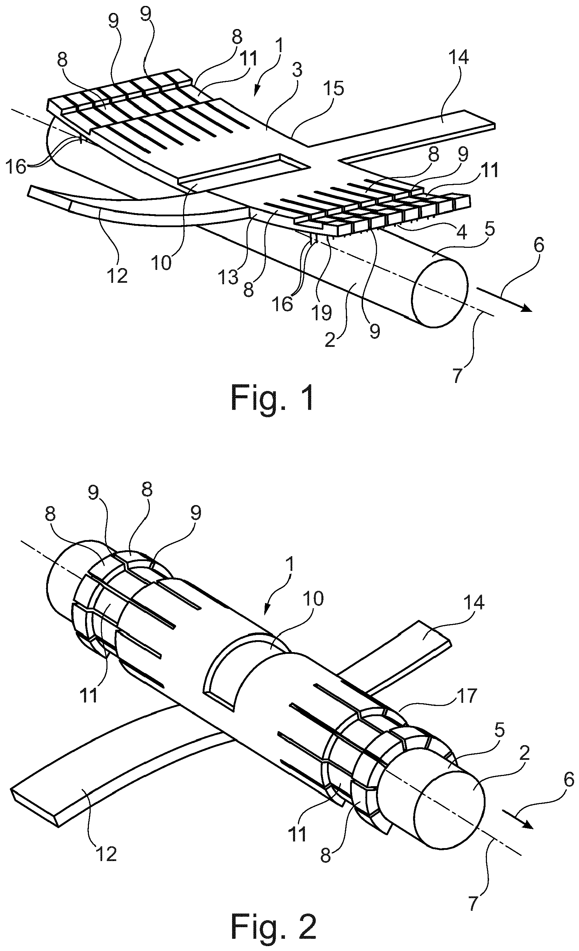

Fig. 1 shows a perspective view of an embodiment of a device 1 for selectively electrically stimulating a nerve 2. The device 1 comprises a substrate 3 which is configured to be arranged at least partially surrounding the nerve 2 so as to define an inner surface 4 of the substrate 3 that faces an outer surface 5 of the nerve 2.

The substrate 3 comprises a plurality of structures 8 comprising a respective gap 9 therebetween. The structures 8 are directed parallel to the longitudinal direction 6 of the substrate 3, so that when the substrate 3 is placed on the nerve 2, the longitudinal direction 6 of the substrate 3 is parallel to the axis 7 of the nerve 2 and that each of the structures 8 are configured to come in contact with the outer surface 5 of the nerve 2.

Each of the plurality of structures 8 comprise a respective central dent 10 and two peripheral dents 11, each proximal to a respective end of the structure 8 along the longitudinal direction 6 of the substrate. The central dent 10 and the peripheral dents 11 provide mechanical stability to the device 1.

The substrate 3 comprises a connection element 12 on a first longitudinal side 13 of the substrate 3 that is arranged perpendicular to the longitudinal direction 6 of the substrate 3. The substrate 3 further comprises a tightening strip 14 on the second longitudinal side 15 of the substrate 3. The tightening strip 14 is perpendicular to the longitudinal direction 6 of the substrate 3 and is arranged opposite to that of the connection element 12. When the substrate 3 is placed on the nerve 2, the tightening strip 14 is configured to tighten the grip of the substrate 3 around the nerve 2.

The device 1 comprises a plurality of electrodes 16, each arranged on the inner surface 4 of the substrate 3. Each electrode 16 is an intraneural electrode 19, that is configured to penetrate the outer surface 5 of the nerve 2 to establish an electric connection with a part of the nerve 2 within the nerve 2.

As shown in Fig. 2, the substrate 3 is configured to be bendable around the longitudinal direction 6 of the device 1, the longitudinal direction 6 being parallel to the axis 7 of the nerve 2, when the device 1 is placed on the nerve 2. The substrate 3 is a nerve cuff 17 comprising a flexible material for wrapping the nerve cuff 17 around the nerve 2. The addition of cuff-like structure improves the stability of the stimulation of the nerve 2, reducing the positional shift of contacts which is normally experienced with intrafascicular electrodes and is a principal cause of unwanted change of functional outcome of the stimulation.

When the substrate 3 is placed on the nerve 2 and is wrapped around the nerve 2, the tightening strip 14 is used to tighten the grip of the substrate 3 around the nerve 2. The device 1 is implanted on the nerve 2 first by tying the tightening strip 14 around the nerve 2 in the mid-plane. At this point the electrodes 16 and the structures 8 are distributed around the circumference of the nerve 2, the tip of the electrodes 16, as shown in Fig. 1, remain touching the outer surface 5 of the nerve 2 but not yet penetrating it. The electrodes 16 and the structures 8 are finally tied tightly around the nerve 2 means of the tightening strip 14. Especially, the central dent 10 enables a surgeon to attach the wound tightening strip 14 in the central dent 10. The design of the electrodes 16, as shown in Fig. 1, is intended so that the electrodes 16 penetrate the nerve 2 radially when tied, with minimal lateral forces which can deviate or bend the electrodes 16 during insertion.

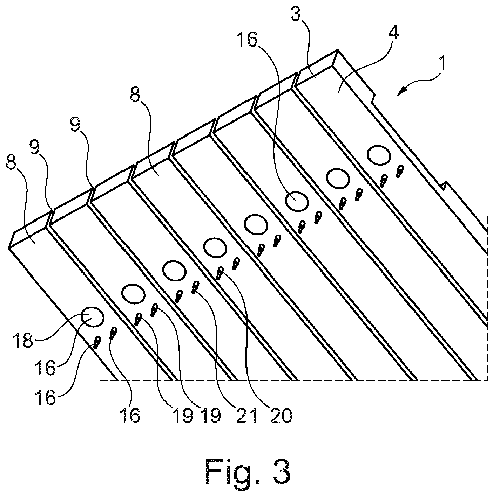

As shown in Fig. 3, the one respective electrode 16 is a surface electrode 18, which is conductive two-dimensional surface pad arranged on the inner surface 4 of each of the structure 8 of the substrate 3 to come in contact with the outer surface 5 of the nerve 2, as shown in Fig. 1 and 2, and configured to establish the electric connection with a part of the nerve on the outer surface 5 of the nerve 2.

Furthermore, two additional electrodes 16, that are intraneural electrodes 19 and that are in the form of spikes are arranged extending from the inner surface 4 of each of the slotted structure 8 of the substrate 3. Each spike comprises a partially insulated electrically conductive material 20, wherein a tip 21 of the spike is non-insulated, so that the electric connection is established with the part of a nerve 2 within the outer surface 5 of the nerve 2, as shown in Fig. 1. Such a concurrent use of electrodes 16 to electrically stimulate at least a part of the nerve 2 on the outer surface 5 of the nerve 2 as well at least a part of the nerve 2 within the nerve 2 enables a selective stimulation of the nerve 2. Three electrodes 16 are arranged on the inner surface 4 of each structure 8 near to one end of each structure 8.

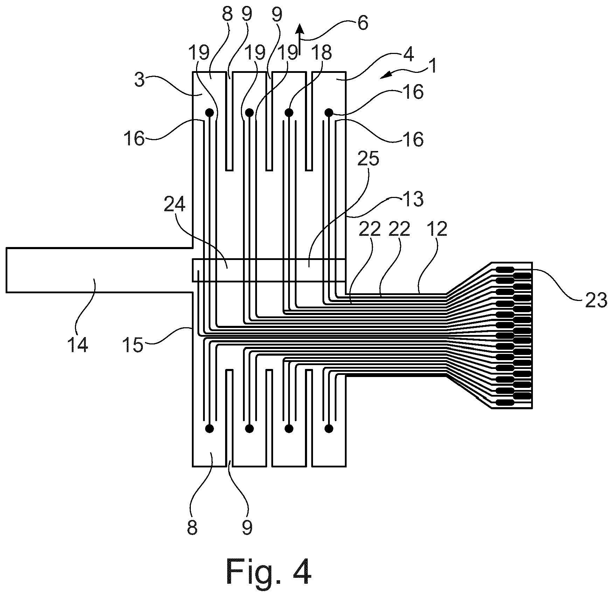

Fig. 4 depicts a schematic view of the inner surface 4 of the device 1. The substrate 3 comprises the connection element 12 on the first longitudinal side 13 of the substrate 3 that is arranged perpendicular to the longitudinal direction 6 of the substrate 3. The substrate 3 further comprises the tightening strip 14 on the second longitudinal side 15 of the substrate 3. The tightening strip 14 is perpendicular to the longitudinal direction 6 of the substrate 3 and is arranged opposite to that of the connection element 12.

The connection element 12 comprises a plurality of at electrically conducting channels 22. Each conducting channel 22 comprises a respective electrode 16, such as one surface electrode 18 and two intraneural electrodes 19 near each end of each side of the structure 8. One end 23 of the conducting channel 22 is connectable to the pulse generator, not shown in figures, which is configured to supply electric current to a part of the nerve 2, as shown in Fig. 1, so that each of the electrodes 16 can be supplied with respective electric currents independent from each other.

The substrate 3 further comprises a central section 24, wherein the inner surface 4 of the substrate 3 corresponding to the central section 24 comprises a central conductive surface pad 25 extending the whole width of the device 1, that is in a direction perpendicular to the longitudinal direction 16 of the substrate 3 but in the plane of the inner surface 4 of the substrate 3.

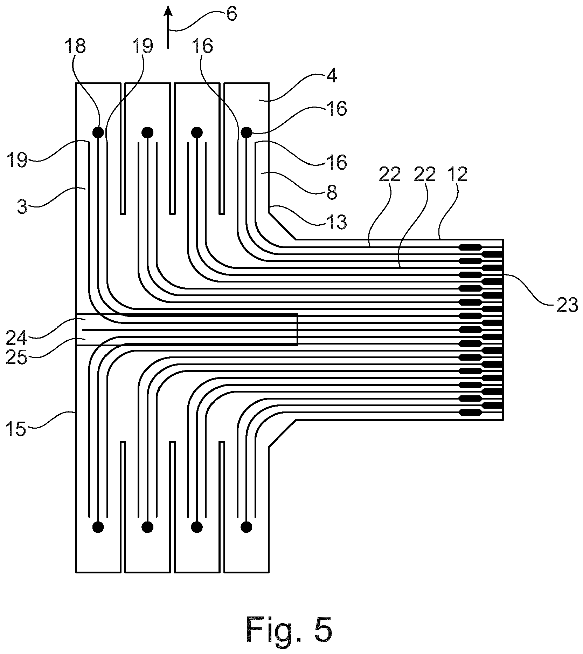

Fig. 5 depicts a schematic view of the inner surface 4 of another embodiment of the device 1 similar to that shown in Fig. 4, wherein the device 1 does not comprise a tightening strip 14, as shown in Fig. 4. Here, the connection element 12 further performs the functionalities of the tightening strip shown in Fig. 4.

The inventive device 1 for selectively electrically stimulating at least one nerve 2 and the method for selectively electrically stimulating at least one nerve by means of the inventive device 1, enable a bioelectronic treatment of diseases by the means of peripheral nerve stimulation, whose therapeutic effects have been demonstrated for several pathologies and its use allowed for clinical practice in multiple instances. The higher selectivity, stability, and/or ease of implant compared to previous neural interfaces from the state of the art, can translate to diminish adverse effect, cost, and ultimately higher adoption.

**List of reference signs**

| | |
|---|---|
| Device | 1 |
| Nerve | 2 |
| Substrate | 3 |
| Inner surface of the substrate | 4 |
| Outer surface of the nerve | 5 |
| Longitudinal direction | 6 |
| Axis of the nerve | 7 |
| Structure | 8 |
| Gap | 9 |
| Central dent | 10 |
| Peripheral dent | 11 |
| Connection element | 12 |
| First longitudinal side of the substrate | 13 |
| Tightening strip | 14 |
| Second longitudinal side of the substrate | 15 |
| Electrode | 16 |
| Nerve cuff | 17 |
| Surface electrode | 18 |
| Intraneural electrode | 19 |
| Insulated metal | 20 |
| Tip of the spike | 21 |
| Conducting channel | 22 |
| One end of the conducting channel | 23 |
| Central section | 24 |
| Central conductive surface pad | 25 |

## Claims

1. Device (1) for electrically stimulating at least one nerve (2), the device comprising:
- a bendable substrate (3) which is configured to be arranged at least partially surrounding the nerve (2) so as to define an inner surface (4) of the substrate (3) that faces an outer surface (5) of the nerve (2); and
- at least two electrodes, wherein at least one of the electrodes is a surface electrode (18) and at least one of the electrodes is an intraneural electrode (19), each arranged on the inner surface (4) of the substrate (3), wherein the at least one surface electrode (18) is configured to establish an electric connection with a part of the nerve (2) on the outer surface (5) of the nerve (2), and wherein the at least one intraneural electrode (19) is configured to penetrate the outer surface (5) of the nerve (2) to establish an electric connection with a part of the nerve (2) within the nerve (2).

2. Device (1) according to claim 1, wherein the intraneural electrode (19) is a spike extending from the inner surface (4) of the substrate (3) and arranged to penetrate the outer surface (5) of the nerve (2) and configured to establish the electric connection with a part of the nerve (2) within the nerve (2), the spike comprising an partially insulated part (20) made of electrically conductive material, wherein a tip (21) of the spike is non-insulated, so that the electric connection is established with the part of a nerve (2) within the nerve (2).

3. Device (1) according to claim 1 or 2, wherein the device (1) is configured to supply electric current via the respective electrode (16) to a respective part of the nerve (2) and/or the device (1) is configured to receive a neuro-signal from a respective part of the nerve (2) via the respective electrode (16).

4. Device (1) according to any of the aforementioned claims, wherein the substrate (3) comprises at least one connection element (12), wherein the connection element (12) comprises at least two electrically conducting channels (22), wherein at least one of the conducting channels is an electric current channel and/or at least one of the conducting channels is a neuro-signal channel, each conducting channel (22) is connected to a respective electrode (16), wherein one end (23) of the electric current channel (22) is connected or is connectable to an electric energy source in order to supply electric current to a part of the nerve (2), and/or wherein one end of the neuro-signal channel (22) is connected or is connectable to a recorder in order to transmit a neuro-signal from a respective part of the nerve.

5. Device (1) according to claim 4, wherein the device (1) comprises a control unit that is configured to adjust a property of the electric current that is transmitted to a respective electrode (16).

6. Device (1) according to claim 5, wherein the device (1) comprises a recorder which is configured to record at least one neuro-signal from at least one part of the nerve (2) and which is connected to the control unit in order to transmit a signal corresponding to the recorded neuro-signal from at least one part of the nerve (2) to the control unit, such that the control unit can adjust a property of the electric current that is transmitted to a respective electrode (16) to stimulate the at least one part of the nerve (2) based on the at least one neuro-signal recorded by the recorder.

7. Device (1) according to any of the aforementioned claims, wherein the substrate (3) comprises a central section (24), wherein the inner surface (4) of the substrate (3) corresponding to the central section (24) comprises a central conductive surface pad (25) extending at least 90% of a width of the central section (24), the width being perpendicular to the longitudinal direction and within the plane of the substrate (3), wherein the longitudinal direction (6) of the substrate (3) is defined to be parallel to the axis (7) of the nerve (2) when the substrate (3) is placed on the nerve (2), wherein the central conductive surface pad (25) is configured to establish the electric connection with a respective part of the nerve (2) on the outer surface (5) of the nerve (2).

8. Device (1) according to claim 7, wherein the device is configured to apply electric current within a frequency range between 4 kHz to 100 kHz via the central conductive surface pad (25), preferably between 10 kHz and 20 kHz, so that a virtual cut of the nerve in a longitudinal direction (6) parallel to the axis (7) of the nerve (2) can be performed, in which a part of the nerve (2) on one side of the central section (24) in the longitudinal direction (6) of the nerve (2) can be treated independent of the part of the nerve (2) on the other side of the central section (24).

9. Device (1) according to any of the aforementioned claims, wherein at least two electrodes (16) are arranged on the inner surface (4) of the substrate (3), wherein each of the at least two electrodes (16) are arranged opposite to each other in a longitudinal direction (6) of the substrate (3), wherein when the substrate (3) is placed on the nerve (2), the longitudinal direction (6) of the substrate (3) is parallel to the axis (7) of the nerve (2), wherein the at least two electrodes are arranged at a predetermined distance from each other, wherein the predetermined distance is at least 75% of the length of the substrate in the longitudinal direction.

10. Device (1) according to any of the aforementioned claims, wherein the substrate (3) comprises at least two structures (8) comprising a gap (9) therebetween, wherein the structures (8) are directed parallel to a longitudinal direction (6) of the substrate (3), so that when the substrate (3) is placed on the nerve (2), the longitudinal direction (16) of the substrate (3) is parallel to the axis (7) of the nerve (2), and that each of the gaps (9) facilitates the bendability of the substrate when the substrate (3) has to be arranged at least partially surrounding the nerve (2).

11. Device (1) according to claim 10, wherein the device comprises at least two structures extending from the central section (24) in opposite directions along the longitudinal direction, wherein at least one electrode (16) is arranged on the inner surface (4) of the structure (8).

12. Device (1) according to claims 10 or 11, wherein one surface electrode (18) and two intraneural electrodes (19) are arranged on the inner surface (4) of the structure (8).

13. Device (1) according to any of the aforementioned claims, wherein the substrate (3) is a nerve cuff (17) comprising a flexible material for wrapping the nerve cuff (17) around the nerve (2).

14. Device (1) according to any of the aforementioned claims, wherein the substrate (3) comprises at least one tightening strip (14), so that when the substrate (3) is placed on the nerve (2), the tightening strip (14) is configured to tighten the grip of the substrate (3) around the nerve (2).

15. Method for electrically stimulating at least one nerve (2) by means of a device (1) according to any of the claims 1 to 14, the method comprising a placing of the bendable substrate (3) on the nerve (2) at least partially surrounding the nerve (2), so that the inner surface (4) of the substrate (4) comes in contact with the outer surface (5) of the nerve (2), wherein at least one surface electrode (18) establishes an electric connection with a part of the nerve (2) on the outer surface (5) of the nerve (2), and wherein at least one intraneural electrode (19) penetrates the outer surface (5) of the nerve (2) to establish an electric connection with a part of the nerve (2) within the nerve (2).
